# EUROPÄISCHE PATENTSCHRIFT

(11) **EP 0 456 122 B1**
(45) Veröffentlichungstag und Bekanntmachung des Hinweises auf die Patenterteilung: **29.11.1995**
(21) Anmeldenummer: 91107165.2
(22) Anmeldetag: 03.05.1991
(51) Int. Cl.: A61N 1/30

(54) **Galvanisch aktives transdermales therapeutisches System**
Galvanic, active, transdermal, therapeutic system
Système thérapeutique actif, galvanique et transcutané

(30) Priorität: 10.05.1990 DE 4014913
(43) Veröffentlichungstag der Anmeldung: 13.11.1991
(73) Patentinhaber: LTS LOHMANN Therapie-Systeme GmbH, 56567 Neuwied (DE)
(72) Erfinder: Horstmann, Michael, Dr., W-5450 Neuwied 1 (DE)
(74) Vertreter: Flaccus, Rolf-Dieter, Dr.

(56) Entgegenhaltungen:
- EP-A- 0 060 451
- EP-A- 0 308 572
- FR-A- 762 259
- FR-A- 2 263 792
- FR-A- 2 299 043
- GB-A- 2 206 493

## Beschreibung

Die Erfindung betrifft ein transdermales therapeutisches System mit schichtförmigem Aufbau flächenförmiger galvanischer Elemente (3,4), die voneinander isoliert angebracht über eine elektrisch leitfähige Verbindungsschicht (2) in Reihe geschaltet sind und deren hautseitige Elektroden leitfähige Schichten (6,7) tragen, wobei der schichtförmige Aufbau durch eine elektrisch isolierende wirkstoffundurchlässige Rückschicht abgedeckt ist, wobei die galvanischen Elemente (3,4) jeweils aus einer pulverisiertes Zink enthaltenden Schicht (8) einer Elektrolytschicht (9) und einer weiteren Schicht (10) aus pulverisiertem Mangandioxid bestehen.

Das Prinzip der transkutanen Iontophorese, das heißt, des Einsatzes galvanischer (elektrostatischer) elektrischer Felder zur therapeutischen Einbringung von ionischen Substanzen in menschliche oder tierische Gewebe ist bereits seit langem bekannt (Pivati 1747, zitiert bei Wearly, L. et al, J. Contr. Release 8, 237-250 (1989)). Solche Systeme sind prinzipiell mindestens aus einer Spannungsquelle und zwei Elektroden aufgebaut, welche voneinander isoliert auf der menschlichen Haut elektrisch leitend aufliegen. Dabei enthält eine der beiden Elektroden einen meist ionischen Arzneistoff, der durch die im elektrischen Feld herrschende Feldstärke in die Haut hinein transportiert wird.

Das Prinzip fand und findet Anwendung zum Beispiel in der experimentellen Pharmakologie, um ionische Substanzen in für diese zunächst schwer zugängliche tierische Gewebe einzubringen (zum Beispiel Mikroiontophorese in bestimmte Gehirnabschnitte). Auch in der klinischen Medizin ergeben sich schon seit langem begrenzte Einsatzgebiete in der Therapie zum Beispiel von übermäßiger Schweißsekretion durch Leitungswasseriontophorese oder auch - im Gegensatz hierzu - in der Funktionsprüfung von Schweißdrüsen durch perkutan iontophoretisch zugeführtes Pilocarpin.
Neben dem limitierten Bereich der Lokalbehandlung wurde die Bedeutung der Iontophorese in den letzten Jahrzehnten zunehmend auch für den Bereich der systemischen perkutanen Arzneibehandlung erkannt.
Die sogenannten transdermalen therapeutischen Systeme sind solche Applikationsformen, welche die gesteuerte Abgabe von Wirkstoffen an den Gesamtorganismus durch die Haut hindurch möglich machen.

Diese Art der Applikation ist gegenüber dem oralen Zufuhrweg beispielsweise durch Vermeidung des first-pass-Effektes und Verlängerung der biologischen Wirkdauer deutlich im Vorteil. Leider erweist sich jedoch der größte Teil der medizinisch verwendbaren Wirkstoffe als nicht ausreichend hautgängig - es handelt sich hier vor allem um ionische Arzneistoffe oder solche mit zu hohem Molekulargewicht. Da mit dem Prinzip der Iontophorese die Passage der Haut gerade für kationische oder anionische Wirkstoffe ermöglicht werden kann, eröffnet sich hier ein therapeutisch außerordentlich interessantes Feld.

Bei den aus der Literatur bekannten Lösungsansätzen ist ein deutlicher Trend zur Miniaturisierung zu erkennen, die bereits damit beginnt, daß man als Spannungsquelle anstelle stationärer Stromquellen eine in die Apparatur eingebaute Batterie verwendet (US-Patent 3,163,166, DE 32 25 748, EPA 0 240 593).

Die Auswahl beziehungsweise Konstruktion einer geeigneten Spannungsquelle für iontophoretische transdermale Systeme ist für Qualität und Preis dieser Geräte in erster Linie entscheidend. Es kommt darauf an, kleine, leichte, aus physiologisch verträglichen Materialien bestehende und preiswerte Batterien zu verwenden. Handelsübliche Zink-Kohle-Trockenbatterien sind schon wegen ihres hohen Gewichtes hierzu nicht geeignet. Quecksilber- und Lithium-Knopfzellen, die bereits wiederholt zum Betrieb in Iontophorese-Systemen vorgeschlagen worden sind, sind zwar relativ leicht, aber teuer. Außerdem enthalten solche Elemente zum Teil für den Menschen toxische Stoffe (Quecksilber etc.), ihre Kapazität ist ferner unnötig hoch, so daß getragene Systeme entweder mit fast voller Batterie weggeworfen werden müssen oder es wird eine konstruktive Trennung zwischen Energiequelle (wiederverwendbar) und Elektroden (Einmalverwendung) erforderlich. In letzterem Fall muß aber in Kauf genommen werden, daß die Handhabung erschwert wird und durch die unterschiedlich lange Lebensdauer solcher Batterien die Wirkstoffzufuhr unvorhersehbar plötzlich beendet sein kann. Hierdurch ist die Sicherheit in der Anwendung gefährdet.

Die Transportrate des pharmakologischen Wirkstoffes in die Haut ist in erster Linie nicht von der Spannung im iontophoretischen Feld abhängig, sondern von der Stromstärke. Nach dem Ohmschen Gesetz kann also ein schwankender Hautwiderstand bei konstanter Versorgungsspannung einen schwankenden iontophoretischen Strom und damit einen unvorhersehbaren Wirkstofffluß verursachen. Aus diesem Grunde wurden Einrichtungen zur Konstanthaltung des elektrischen Stromes vorgeschlagen (z.B. EPA 0 254 965).

Auch bei der Verwendung gleichbleibender Stromstärke treten Störeffekte auf, die vor allem bei hoher Stromdichte den Wirkstofffluß in die Haut verändern; so kommt es durch Wanderung von Wasserstoff- und Hydroxylionen zu einer pH-Verschiebung mit Einfluß auf den Anteil ionisch vorliegenden Wirkstoffes oder es verändern sich durch unterschiedliche Wanderungsgeschwindigkeit die Konzentrationsverhältnisse zwischen Wirkstoffionen und Begleitionen aus der Umgebung (Konkurrenzionen).

Auch diese Erscheinungen versuchte man, u.a. durch elektronisch gesteuerte Pulsationen des elektrischen Stromes, zu beseitigen (z.B. EP-A-0 060 451).
Zusatzeinrichtungen der vorgenannten Art (Stromkonstanthaltung, Pulsation) lassen sich zwar heute auf vergleichsweise kleinem Raum realisieren, aber sie machen solche Systeme ganz erheblich komplizierter und teurer.
Mit besonderen räumlichen Anordnungen befaßt sich z.B. die EPA 0 278 473, mit speziellen hautseitigen Elektroden z.B. EPA 0 269 246, EPA 0 252 732, EPA 0 182 520, welche in erster Linie widerum einen kompakteren Aufbau zum Ziel haben.

Aus dem Dokument GB-A2 206 493 ist ein gattungsgemäßes perkutanes oder perunguales System zur Administration einer gelösten oder teilweise gelösten Substanz zur Iontophorese an einen Organismus bekannt. Das System besteht in schichtförmigen Aufbau aus zwei Streifen mit jeweils einer flächenförmigen Metallschicht mit einer den Wirkstoff enthaltenden Auflage sowie einer isolierenden Rückschicht. Die aus den Metallschichten gebildeten galvanischen Elemente sind über einen elektrisch leitfähigen Draht miteinander verbunden.
Das Dokument FR-A-2 299 043 offenbart eine Vorrichtung zur Ionisation des Zellsystems eines Lebewesens mit einem isolierten Behälter oder Träger einer Materie oder einem elektrisch isolierten Metall für 2 oder mehre Elemente aus Metall oder leitfähiger Legierung, wobei diese durch ein schwammförmiges Material verbunden sind, welches mit dem zu ionisierenden Produkt getränkt sind.

Der Erfindung liegt die Aufgabe zugrunde, ein iontophoretisches transdermales therapeutisches System mit einer geeigneten Spannungsquelle bereitzustellen, welches klein und leicht ist, darüber hinaus kostengünstig zu fertigen, bequem und sicher in der Anwendung ist und in seiner Gesamtheit aus physiologisch unbedenklichen Stoffen aufgebaut ist. Die Lösung der Aufgabe gelingt bei einem transdermalen therapeutischen System mit schichtenförmigem Aufbau flächenförmiger galvanischer Elemente entsprechend dem Oberbegriff von Anspruch 1 mit der Erfindung dadurch, daß die den Kontakt vermittelnde Außenfläche zumindest einer der Elektroden eine zusätzliche Leitschicht trägt, die pulverisierten Kohlenstoff enthält, daß zur Vermeidung von Kurzschlüssen oder Lokalelementbildung zwischen den galvanischen Elementen und den leitfähigen Schichten eine mit Aussparungen versehene Isolierschicht sowie eine zusätzliche Isolierschicht zwischen den galvanischen Elementen und der Verbindungsschicht eingebracht ist, und daß den aus feuchtigkeitsaufnehmendem und nach Feuchtigkeitszutritt ionisch leitfähigem Material bestehenden Schichten, von welchen eine einen pharmazeutischen Wirkstoff enthält, das bei der Herstellung verwendete Wasser durch Trocknung nahezu vollständig entfernt ist.

Weitere vorteilhafte Ausgestaltungen der Erfindung sind entsprechend den Merkmalen der abhängigen Ansprüche vorgesehen.

Hierbei wird die Strom-Zeit-Kennlinie der Batterie selbst zur Steuerung der Zufuhrgeschwindigkeit des Wirkstoffes eingesetzt. Dabei besteht einerseits die Möglichkeit, bei entsprechend großer Kapazität der Spannungsquelle eine gleichmäßige Stromstärke und damit einen konstanten Wirkstofffluß zu erzeugen. Andererseits kann der abfallende Innenwiderstand einer mit geringer Kapazität ausgestatteten Spannungsquelle zur Erzeugung nach und nach abfallender Stromstärke und damit geringeren transdermalen Wirkstoffflusses dienen. Ferner besteht die Möglichkeit, das transdermale therapeutische System feuchtigkeitsaktivierbar zu gestalten, so daß der mit zeitlicher Verzögerung einsetzende elektrische Strom den Wirkstofffluß mit vorherbestimmter zeitlicher Verzögerung in Gang setzt und aufrechterhält. Im Gegensatz zu EPA 0 282 982, in welcher ein "trockenes" transdermales Iontophoresesystem die Verlagerung des Stromflusses über wassergefüllte "shunts" zum großflächigeren stratum corneum bewirkt, kann im erfindungsgemäßen Gerät wahlweise ein durch transepidermalen Wasserverlust feuchter werdendes Milieu auf der Haut zur Aktivierung der Energiequellen dienen.
Der Aufbau der erfindungsgemäßen galvanischen Zellen (3,4) entspricht im Grundprinzip dem seit Jahrzehnten in Trockenbatterien bewährten Leclanche-Element, welches auch in diversen flächenförmigen Varianten bekannt ist (z.B. JP 62 128 447).
Grundsätzlich besteht die Minuselektrode (8) des erfindungsgemäßen Elementes aus einer Dispersion von Zinkpulver mit einer bevorzugten Korngröße von 0,5 bis 50 Mikrometern, welches sich in einer die Teilchen verbindenden polymerhaltigen Schicht befindet. Als Polymere sind z.B. Polyacrylsäure und ihre Ester, Polyisobutylen, Polyvinylacetat und Copolymere sowie in ihrer Funktion vergleichbare andere Materialien geeignet. Auch können Additive zur Abstimmung der Härte oder klebrigmachende Zusätze sinnvoll sein, um die Flexibilität und die Verklebung mit den Nachbarschichten zu verbessern. Es sind ebenfalls Zusätze in Höhe von bevorzugterweise 1 bis 20 Gewichtsprozenten Kohle oder Graphit möglich, welche der Verbesserung der Leitfähigkeit zwischen den Zinkpartikeln dienen können. Der Zinkanteil liegt vorzugsweise bei 60 bis 95 Gewichtsprozenten.
Die Pluselektrode (10) besteht aus einer gemeinsamen Dispersion von Mangandioxid und Kohle beziehungsweise Graphit in einem Polymer oder einer polymerhaltigen Masse entsprechend der bei der Minuselektrode (8) beschriebenen. Der Gewichtsanteil der anorganischen Bestandteile kann bei 40 bis 95 Gewichtsprozenten liegen. Im allgemeinen überwiegt mengenmäßig das Mangandioxid gegenüber dem Graphit, dies ist jedoch keine notwendige Bedingung.

An die Elektrolytschicht (9) werden die Anforderungen der ionischen Leitfähigkeit und der Vermeidung von Kurzschlüssen, also mechanischer Festigkeit gestellt. Zur Erfüllung dieser Anforderungen stehen dem Fachmann eine Reihe von Möglichkeiten zu Gebote.
So können zur Erhöhung der Leitfähigkeit ionische Substanzen zugesetzt sein, wie zum Beispiel anorganische Salze, besonders bevorzugt Ammoniumchlorid oder viskositätssteigernde Substanzen wie z.B. Stärke oder Polyvinylalkohol. Als Strukturkomponenten sind Vliesstoffe, Papiere verschiedener Qualitäten und Dicken, poröse Kunststofffolien oder hochkonzentrierte Pasten aus im wesentlichen inerten Stoffen (z.B. Gips) einsetzbar.
Die Dicke der drei einzelnen Schichten der Elemente ist für die Funktion nicht ausschlaggebend; sie kann vorzugsweise zwischen einen und fünfhundert Mikrometern liegen.

Die Herstellung der galvanischen Elemente kann Schicht für Schicht durch Lösen bzw. Suspendieren in einem geeigneten Lösemittel, Ausstreichen in dünner Schicht und anschließendes Trocknen geschehen, gefolgt von einer Kaschierung der drei Schichten aufeinander. Es ist ebenfalls möglich, Pluselektrode und/oder Minuselektrode unter Verwendung einer thermoplastischen Polymermischung in einem Heißschmelzverfahren auszustreichen, zu extrudieren oder zu walzen. Die Elekrolytschicht kann auch durch Imprägnieren eines Papieres, Vlieses oder einer porösen Folie mit der Lösung eines oder mehrerer Elektrolyten entstehen.
Sofern die Leitfähigkeit von Anode und Kathode nicht ausreicht, können die Elemente auf den außen liegenden Flächen mit einer zusätzlichen leitfähigen Schicht (12) überzogen sein, deren anorganische Komponente Kohlenstoff ist. Als Bindemittel eignen sich die gleichen Polymermischungen wie für Kathode und Anode der Elemente beschrieben.
Die hautseitigen Elektroden mit den leitfähigen Schichten (6,7) des transdermalen therapeutischen Systems befinden sich bündig oder leicht überlappend unmittelbar auf der Kathode beziehungsweise Anode der in Serie geschalteten galvanischen Elemente.
Sie bestehen aus einem porösen, wasserdampfdurchlässigen und wasseraufnehmenden Vlies, Papier, Gel oder einer Folie, die mit Wirkstoff, Elekrolyten und gegebenenfalls weiteren Stoffen dotiert sein können. Kationische Wirkstoffe werden vorzugsweise unter dem Pluspol, anionische unter den Minuspol gebracht.

Auf der hautabgewandten Seite sind die beiden galvanischen Zellen über eine elektrisch leitfähige Verbindungsschicht (2), z.B. aus Aluminium, aluminiumbedampfter Folie oder einem Material entsprechend der oben erfolgten Beschreibung optionaler zusätzlicher leitfähiger Schichten miteinander leitend verbunden.

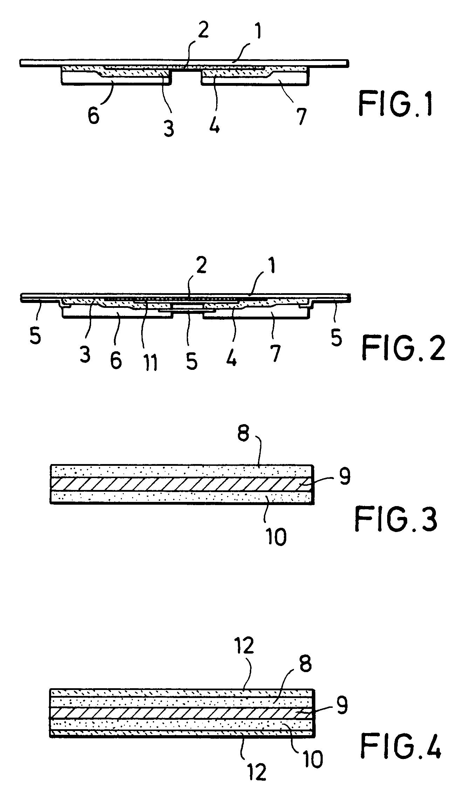

Um Kurzschlüsse zwischen hautseitigen Elektroden und dem elektrischen Potential der Verbindungsschicht auszuschließen, ist als Maske eine Isolierschicht (5), ggf. noch eine weitere Schicht (11) entsprechend Fig. 2 aufgebracht. Diese kann aus beliebigen physiologisch verträglichen Materialien mit ausreichender elektrischer Isolierwirkung bestehen, z.B. aus Polyisobutylen, Polyethylen, Polyethylenterephthalat, Siliconkautschuk oder Polyvinylacetat und seinen Copolymeren.
Um das therapeutische System auf der Haut zu fixieren, können Isolierschicht und Rückschicht haftklebend ausgerüstet sein. Dies kann u.a. durch vorheriges Aufbringen einer elektrisch im wesentlichen nicht leitfähigen Schicht aus Polyacrylaten, Kautschuk-Harz-Mischungen und funktionsgleichen Stoffen geschehen.
Zum Schutz des Systems kann es vor Gebrauch mit einer dehäsiv ausgerüsteten Schutzfolie hautseitig abgedeckt sein. Wahlweise kann bei der Herstellung der erfindungsgemäßen Vorrichtung verwendetes Wasser durch Trocknung nahezu vollständig entfernt werden, um die Lagerfähigkeit zu erhöhen. Dabei erfolgt die Aufbewahrung vorzugsweise in einer hierzu geeigneten wasserdampfundurchlässigen Verpackung (z.B. aluminisierter Verbundsiegelbeutel).

Vor Gebrauch wird diese Ausführungsform des Erfindungsgegenstandes durch kurzzeitiges Einbringen in eine wassergesättigte Atmosphäre oder durch Eintauchen in Wasser aktiviert. Durch Diffusion gelangt Feuchtigkeit durch die verwendeten Polymere auch in die Elektrolytschichten der galvanischen Zellen. Besonders schonend verläuft die Aktivierung durch nach dem Aufkleben des noch trockenen Systems eindringenden Wasserdampf aus der Haut (Okklusivwirkung/perspiratio insensibilis).

Ein wesentlicher Vorteil des Systems liegt darin, daß die Stromstärke und damit der Wirkstofffluß durch den systembedingten Innenwiderstand reproduzierbar festgelegt ist und durch entsprechende Gestaltung der Zellen den therapeutischen Erfordernissen angepaßt werden kann. Ist zum Beispiel eine während des Tragens bereits sanft abfallende Freisetzungsrate erwünscht, werden die Elektroden der galvanischen Elemente sehr dünn ausgelegt, so daß durch den sukzessiven Verbrauch an aktivem Elektrodenmaterial der iontophoretische Strom in vorherbestimmter und reproduzierbarer Weise abfällt. Dies wäre u.a. auch für eine tageszeitgerechte Therapie nutzbar.

Insgesamt bedeutet der erfindungsgemäße Aufbau eine ganz wesentliche Vereinfachung, da elektrische Steuerungseinrichtungen etc. entfallen. Das Gerät ist somit preiswerter herstellbar und bequemer auf der Haut zu tragen als Systeme nach dem Stand der Technik.

Die Figuren 1 bis 4 zeigen den Gesamtaufbau des erfindungsgemäßen Systems. Es bedeuten: 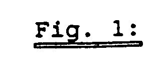
- 1: - wirkstoffundurchlässige Rückschicht
- 2: - elektrisch leitfähige Verbindungsschicht
- 3,4: - flächenförmige galvanische Elemente
- 6,7: - optional wirkstoffhaltige ionisch leitfähige Schichten

### Fig. 2:

- 1: - wirkstoffundurchlässige Rückschicht
- 2: - elektrisch leitfähige Verbindungsschicht
- 3,4: - flächenförmige galvanische Elemente
- 5: - mit Aussparungen versehene Isolierschicht
- 6,7: - optional wirkstoffhaltige ionisch leitfähige Schichten
- 11: - weitere Isolierschicht

### Fig. 3:

- 8: - pulverisiertes Zink enthaltende Schicht
- 9: - Elektrolytschicht
- 10: - Mangandioxid und Kohlenstoff enthaltende Schicht

### Fig. 4:

- 8: - pulverisiertes Zink enthaltende Schicht
- 9: - Elektrolytschicht
- 10: - Mangandioxid und Kohlenstoff enthaltende Schicht
- 12: - Leitschicht, pulverisierten Kohlenstoff enthaltend

Die Erfindung wird anhand der nachfolgenden Beispiele erläutert: 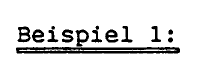
Herstellung eines flächigen galvanischen Elementes:
- Phase 1: : 100 g Zink, fein gepulvert
15 g Graphit, fein gepulvert
10 g Copolymeres aus Vinylacetat und Vinyllaurat (Vinnapas B500/40VL (Wacker))
werden in 20 g Butanon-2 gelöst bzw. suspendiert.
- Phase 2: : 80 g Mangandioxid, fein gepulvert
25 g Graphit, fein gepulvert
10 g Copolymeres aus Vinylacetat und Vinyllaurat (Vinnapas B500/40VL (Wacker))
werden in 60 g Butanon-2 ebenfalls suspendiert.

Feines Schreibpapier (60 g/m²) wird mit einer Lösung aus
3,8 g Ammoniumchlorid
und 1,0 g Polyvinylpyrrolidon
in 20 ml Wasser imprägniert, so daß nach Trocknung ein Flächengewicht von ca. 90 g/m² resultiert.

Das Papier wird mit einer Schicht der Phase 1 einer Dicke von 200 Mikrometern überzogen. Nach Trocknung wird das gleiche Papier auf der anderen Seite mit einer Schicht der Phase 2 einer Dicke von 300 Mikrometern überzogen.

Das Element wird bei 100°C zehn Minuten lang getrocknet und anschließend vor Feuchtigkeit geschützt gelagert.

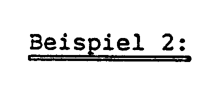

Herstellung eines galvanisch aktiven transdermalen therapeutischen Systems: (siehe hierzu Fig. 2)
Ein 25 x 25 mm messendes Stück Aluminiumfolie (2) (Dicke ca. 10 Mikrometer) wird mittig auf ein 50 x 50 mm großes Stück mit 40 g/m² Durotak 280-2516, einem klebrigen Acrylat-Copolymer (National Starch & Chemical), klebend ausgerüsteter Polyesterfolie (25 Mikrometer) als Rückschicht (1) geklebt.
Ein 6 x 50 mm großer Klebstreifen (11) aus dem gleichen Material wie (1) wird so auf die Aluminiumschicht geklebt, daß das Quadrat in zwei gleichgroße Rechtecke unterteilt wird.
Aus dem nach Beispiel 1 hergestellten flächenförmigen Material werden zwei Stücke von je 27 x 30 mm geschnitten. Diese Stücke (3 und 4) werden nach Fig. 2 so positioniert, daß die beiden leitenden Aluminiumflächen überlappend bedeckt sind, ohne daß die beiden Elemente einander berühren. Dabei weist bei einem Element die Zinkschicht, bei dem anderen die Mangandioxidschicht nach oben.
Entsprechend der Abbildung wird der energieliefernde Teil mit einer Maske (5) aus 60 Mikrometer dicker, klebriger Weichfolie aus Durotak 280-2516 abgedeckt. Auf dieser Schicht verankernd, werden schließlich die beiden 27 x 30 mm großen Vliesträger (6 und 7) befestigt. Der auf der Zinkfläche zu positionierende Vliesträger wurde zuvor mit 0,1 ml 1%iger Epinephrin-hydrochlorid-Lösung beträufelt und schonend bei Raumtemperatur getrocknet.
Das kontralaterale Vlies wird entsprechend mit 0,1 ml 1%iger Natriumdihydrogenphosphat-Lösung betropft und getrocknet.

## Patentansprüche

1. Transdermales therapeutisches System mit schichtförmigem Aufbau flächenförmiger galvanischer Elemente 3,4, die von einander isoliert angebracht über eine elektrisch leitfähige Verbindungsschicht 2 in Reihe geschaltet sind und deren hautseitige Elektroden leitfähige Schichten 6,7 tragen, wobei der schichtförmige Aufbau durch eine elektrisch isolierende wirkstoffundurchlässige Rückschicht 1 abgedeckt ist, wobei die galvanischen Elemente 3,4 jeweils aus einer pulverisiertes Zink enthaltenden Schicht 8, einer Elektrolytschicht 9 und einer weiteren Schicht 10 aus pulverisiertem Mangandioxid bestehen, dadurch gekennzeichnet, daß die den Kontakt vermittelnde Außenfläche zumindest einer der Elektroden 8,10 eine zusätzliche Leitschicht 12 trägt, die pulverisierten Kohlenstoff enthält, daß zur Vermeidung von Kurzschlüssen oder Lokalelementbildung zwischen den galvanischen Elemente 3,4 und den leitfähigen Schichten 6,7 eine mit Aussparungen versehene Isolierschicht 5 sowie eine Isolierschicht 11 zwischen den galvanischen Elementen 3,4 und der Verbindungsschicht 2 eingebracht ist, und daß den aus feuchtigkeitsaufnehmendem und nach Feuchtigkeitszutritt ionisch leitfähigem Material bestehenden Schichten 6,7, von welchen eine einen pharmazeutischen Wirkstoff enthält, das bei der Herstellung verwendete Wasser durch Trocknung nahezu vollständig entfernt ist.

2. Transdermales therapeutisches System nach Anspruch 1, dadurch gekennzeichnet, daß die flächenförmigen galvanischen Elemente 3,4 mit umgekehrter Anordnung von Anode und Katode im gleichen Abstand zur Oberfläche der Rückschicht 1 angeordnet sind.

3. Transdermales therapeutisches System nach Anspruch 1 oder 2, dadurch gekennzeichnet, daß kationische Wirkstoffe unter dem Pluspol und anionische Wirkstoffe unter dem Minuspol eingebracht sind.

4. Transdermales therapeutisches System nach einem oder mehreren der Ansprüche 1-3, dadurch gekennzeichnet, daß das Grundmaterial der Elektroden und der Leitschicht aus einer oder mehrerer der Komponenten Polyvinylacetat und Copolymere, Polyacrylsäure und Copolymere ihrer Ester, Polyurethan oder Polyisobutylen besteht.

5. Transdermales therapeutisches System nach einem oder mehreren der Ansprüche 1-4, dadurch gekennzeichnet, daß das System durch Zutritt von Hautfeuchtigkeit nach dem Aufkleben auf die Haut aktivierbar ist.

6. Transdermales therapeutisches System nach einem oder mehreren der Ansprüche 1-5, dadurch gekennzeichnet, daß es als Wirkstoff ein Antirheumatikum, an Antiasthmatikum, ein Antidiabetikum oder Antihypertensivum enthält.

7. Verfahren zur Herstellung eines transdermalen therapeutischen System nach einem der vorangehenden Ansprüche, dadurch gekennzeichnet, daß die galvanischen Elemente Schicht für Schicht durch Lösen bzw. Suspendieren in einem geeigneten Lösungsmittel, Ausstreichen in dünner Schicht und Trocknen und anschließende Kaschierung der drei Schichten aufeinander gebildet werden.

8. Verfahren zur Herstellung eines transdermalen therapeutischen Systems nach einem der Ansprüche 1 bis 7, dadurch gekennzeichnet, daß die Elektroden unter Verwendung einer thermoplastischen Polymermischung in einem Heißschmelzverfahren ausgestrichen, extrudiert oder gewalzt werden.

9. Verfahren nach Anspruch 7 oder 8, dadurch gekennzeichnet, daß die Elektrolytschicht der galvanischen Elemente durch Imprägnieren eines Papieres, Vlieses oder einer porösen Folie mit der Lösung eines oder mehrerer Elektrolyte erhalten wird.

10. Verwendung eines transdermalen therapeutischen Systems nach einem der Ansprüche 1 bis 6 oder des Verfahrensproduktes nach einem der Ansprüche 7 oder 8, dadurch gekennzeichnet, daß die Aktivierung durch kurzzeitiges Einbringen vor Gebrauch in eine wassergesättigte Atmosphäre oder durch Eintauchen in Wasser erfolgt.

11. Verwendung des transdermalen therapeutischen Systems nach einem der Ansprüche 1 bis 6 oder des Verfahrensproduktes nach einem der Ansprüche 7 oder 8, dadurch gekennzeichnet, daß die Aktivierung nach dem Aufkleben des noch trockenen Systems durch aus der Haut eindringenden Wasserdampf erfolgt.

## Claims

1. A transdermal therapeutic system with a laminated structure comprising sheet-like galvanic elements 3,4 positioned such that they are insulated from each other and connected in series via an electrically conductive connecting layer 2, the dermad electrodes of said galvanic elements having conductive layers 6,7 thereon, whereby the laminated structure is covered by an electrically insulating, active substance-impermeable backing layer (1), whereby each of the galvanic elements 3,4 consists of a layer 8 containing pulverized zinc, an electrolytic layer 9, and a further layer of pulverized manganese dioxide, characterized in that the outer surface of at least one of the electrodes 8,10, which surface establishes the contact, has an additional conductive layer 12 containing pulverized carbon, that, in order to prevent short circuits or local element formation, an insulating layer 5 provided with recesses is positioned between the galvanic elements 3,4 and the conductive layers 6,7, and an insulating layer 11 is positioned between the galvanic elements 3,4 and the connecting layer 2, and that the water used in the production process is almost completely removed from the layers 6,7 by drying, said layers 6,7 consisting of moisture-absorbing and - after moisture admission - ionically conductive material, one of which layers containing a pharmaceutical active agent.

2. The transdermal therapeutic system according to claim 1 characterized in that the sheet-like galvanic elements 3,4 in reversed arrangement of anode and cathode are positioned at equal distance to the surface of the backing layer 1.

3. The transdermal therapeutic system according to claim 1 or 2 characterized in that cationic active substances are placed under the positive pole and anionic active substances are placed under the negative pole.

4. The transdermal therapeutic system according to one or more of claims 1 to 3 characterized in that the base material of the electrodes and the conductive layer consists of one or more of the components polyvinyl acetate and copolymers, polyacrylic acid and copolymers of the esters thereof, polyurethane, or polyisobutylene.

5. The transdermal therapeutic system accord ing to one or more of claims 1 to 4 characterized in that the activation thereof may be effected by the admission of cutaneous moisture after the system has been placed on the skin.

6. The transdermal therapeutic system according to one or more of claims 1 to 5 characterized in that it contains as active substance an antirheumatic, antiasthmatic, antidiabetic, or antihypertensive.

7. A process for the production of a transdermal therapeutic system as defined in any one of the preceding claims, characterized in that the galvanic elements are formed layer by layer by either dissolution or suspension in a suitable solvent, spreading as thin layer, drying, and subsequent lamination of the three layers on top of each other.

8. A process for the production of a transdermal therapeutic system according to any one of claims 1 to 7, characterized in that the electrodes are spread, extruded, or rolled out in a hot-melt process, using a thermoplastic polymeric mixture.

9. The process according to claim 7 or 8 characterized in that the electrolytic layer of the galvanic elements is obtained by impregnating a paper, non-woven, or porous film, foil or sheet with the solution of one or more electrolytes.

10. The use of a transdermal therapeutic system as defined in any one of claims 1 to 6, or of the process product obtained according to claim 7 or 8, characterized in that the activation is effected by short-time insertion into a water-saturated atmosphere prior to use, or by dipping into water.

11. The use of the transdermal therapeutic system as defined in any one of claims 1 to 6 or of the process product obtained according to claim 7 or 8, characterized in that the activation is effected, after placing the system on the skin in dry condition, by means of water vapor from the skin penetrating into the system.

## Revendications

1. Système thérapeutique transdermique, avec une structure en couches d'éléments galvaniques aplatis (3, 4) qui, isolés l'un de l'autre, sont branchés en série par l'intermédiaire d'une couche électriquement conductrice de liaison (2), et dont les électrodes situées du côté de la peau portent une couche conductrice (6,7), tandis que la structure en couches est recouverte par une couche dorsale, électriquement isolante et imperméable aux produits actifs, les éléments galvaniques (3, 4) étant chacun constitués d'une couche (8), contenant du zinc pulvérisé, d'une couche d'électrolyte (9) et d'une autre couche en dioxyde de manganèse pulvérisé (10), caractérisé en ce que la surface externe d'au moins une des électrodes (8, 10) fournissant le contact porte une couche conductrice supplémentaire (12) qui contient du carbone pulvérisé, en ce que, pour éviter des court-circuits ou la formation locale d'éléments, entre les éléments galvaniques (3, 4) et les couches conductrices (6, 7), une couche isolante (5) munie de découpes et une couche isolante (11) supplémentaires sont installées entre les éléments galvaniques (3, 4) et la couche de liaison (2), et en ce que l'eau utilisée lors de la fabrication est pratiquement complètement éliminée par séchage des couches (6, 7) constituées d'un matériau reprenant l'humidité et devenant ioniquement conducteur après la pénétration d'humidité, une de ces couches contenant un produit pharmaceutiquement actif.

2. Système thérapeutique transdermique selon la revendication 1, caractérisé en ce que les éléments galvaniques plats (3, 4) sont disposés dans un agencement inversé de l'anode et de la cathode, à la même distance par rapport à la surface de la couche dorsale (1).

3. Système thérapeutique transdermique selon la revendication 1, caractérisé en ce que des produits actifs cationiques sont placés sous le pôle positif, tandis que des produits actifs anioniques sont placés sous le pôle négatif.

4. Système thérapeutique transdermique selon une ou plusieurs des revendications 1-3, caractérisé en ce que le matériau de base des électrodes et de la couche conductrice est constitué d'un ou plusieurs des composants tels que, acétate de polyvinyle et ses copolymères, acides polyacryliques et copolymères de leurs esters, polyuréthane ou polyisobutylène.

5. Système thérapeutique transdermique selon une ou plusieurs des revendications 1-4, caractérisé en ce que le système peut être activé par pénétration de l'humidité de la peau, après collage sur la peau.

6. Système thérapeutique transdermique selon une ou plusieurs des revendications 1-5, caractérisé en ce qu'il contient comme produit actif un anti-rhumatismes, un anti-asthme, un antidiabétique ou un anti-hypertension.

7. Procédé de fabrication d'un système thérapeutique transdermique selon l'une quelconque des revendications précédentes, caractérisé en ce que les éléments galvaniques sont fabriqués couche par couche, par mise en solution ou en suspension dans un solvant approprié, étalement en couche mince et ensuite séchage, avec ensuite lamification des trois couches l'une sur l'autre.

8. Procédé de fabrication d'un système thérapeutique transdermique selon l'une quelconque des revendications 1 à 7, caractérisé en ce que les électrodes sont étalées, extrudées ou laminées, par un procédé de fusion à chaud, en recourant à un mélange de polymères thermoplastiques.

9. Procédé selon la revendication 7 ou 8, caractérisé en ce que la couche d'électrolyte des éléments galvaniques est obtenue par imprégnation d'un papier, d'un feutre ou d'une feuille poreuse par la solution d'un ou de plusieurs électrolytes.

10. Utilisation d'un système thérapeutique transdermique selon l'une quelconque des revendications 1-6 ou du produit du procédé selon l'une quelconque des revendications 7 ou 8, caractérisée en ce que l'activation s'effectue par insertion de courte durée, avant utilisation, dans une atmosphère saturée en eau, ou par immersion dans l'eau.

11. Utilisation d'un système thérapeutique transdermique selon l'une quelconque des revendications 1-6 ou du produit du procédé selon l'une quelconque des revendications 7 ou 8, caractérisée en ce que l'activation s'effectue après collage du système encore sec, par la vapeur d'eau pénétrant à partir de la peau.
